Europäisches Patentamt

European Patent Office

Office européen des brevets

(11) Numéro de publication: **0 338 886**
**A1**

# (12) DEMANDE DE BREVET EUROPEEN

(21) Numéro de dépôt: **89400952.1**

(22) Date de dépôt: **06.04.89**

(51) Int. Cl.⁴: **A 61 B 10/00**

(30) Priorité: **18.04.88 FR 8805100**

(43) Date de publication de la demande:
**25.10.89 Bulletin 89/43**

(84) Etats contractants désignés:
**AT BE CH DE ES FR GB GR IT LI LU NL SE**

(71) Demandeur: **Muraz, Pierre**
**321 avenue d'Argenteuil**
**Bois Colombes (Hauts de Seine) (FR)**

**Sultan, Bernard**
**1 à 3 rue Solférino**
**Colombes (Hauts de Seine) (FR)**

(72) Inventeur: **Sultan, Bernard**
**1 à 3, rue Solférino**
**Colombes Hauts de Seine (FR)**

(74) Mandataire: **Cabinet Pierre HERRBURGER**
**115, Boulevard Haussmann**
**F-75008 Paris (FR)**

Une requête en rectification de la figure 1 a été présentée conformément à la règle 88 CBE. Il est statué sur cette requête au cours de la procedure engagée devant la division d'examen (Directives relatives à l'examen pratiqué à l'OEB, A-V, 2.2).

(54) **Récipient stérile pour recueillir des échantillons biologiques à des fins d'analyse.**

(57) Dispositif destiné à faciliter le prélèvement urinaire tout en éliminant les risques de contamination de l'échantillon biologique ainsi recueilli.

Dispositif caractérisé en ce qu'il se compose d'un flacon (100) à extrémité supérieure ouverte (101), coiffée par un premier moyen de fermeture (103, 107) et un fond fermé (102), coiffé par un second moyen de fermeture (104), le second moyen de fermeture (104) étant destiné à coiffer l'extrémité supérieure ouverte (101) après l'enlèvement du premier moyen de fermeture (103, 107) et introduction de l'échantillon biologique dans le flacon (100).

FIG.1

EP 0 338 886 A1

Description

## "Récipient stérile pour recueillir des échantillons biologiques à des fins d'analyse".

La présente invention a pour objet un récipient stérile facilitant le recueil d'un échantillon biologique essentiellement urinaire, tout en limitant considérablement la contamination de ce dernier par d'éventuels germes extérieurs, dits "de souillure".

A l'heure actuelle, le prélèvement urinaire en vue d'un examen cytobactériologique s'effectue dans 95 % des cas au domicile du patient qui récolte lui-même l'échantillon urinaire dans un simple flacon muni d'un bouchon à vis, et l'apporte au laboratoire d'analyse.

Les inconvénients d'utiliser un tel flacon sont multiples :
- Au niveau de la technique de prélèvement, la main tenant le flacon sera presque toujours souillée par le jet urinaire car l'échantillon doit être prélevé au milieu de la miction.
- Au niveau de l'étude cytobactériologique, l'interprétation des résultats sera très souvent faussée par la contamination indirecte de l'échantillon urinaire, dont les causes isolées ou associées sont essentiellement :
- Le toucher ou l'effleurement digital des faces internes stériles du bouchon mais aussi et surtout du bord libre de l'ouverture du flacon.
- Le dépôt provisoire du bouchon durant le remplissage du flacon, sur un quelconque support non stérile.
- La chute accidentelle et fréquente du bouchon.

L'exposition prolongée de ses faces intérieures stériles à l'air libre non stérile et ce pendant toute la durée de la miction et encore davantage si le bouchon est momentanément égaré, ce qui est loin d'être rare.

La présente invention a pour but de remédier à ces inconvénients et se propose de créer un moyen permettant d'améliorer la technique de prélèvement, d'assurer la qualité de l'échantillon biologique pour permettre une meilleure interprétation des résultats de l'analyse biologique.

A cet effet, l'invention concerne un dispositif du type ci-dessus, caractérisé en ce qu'il se compose d'un flacon à extrémité supérieure ouverte, coiffée par un premier moyen de fermeture à fond fermé, coiffé par un second moyen de fermeture, le second moyen de fermeture étant destiné à coiffer l'extrémité supérieure ouverte après l'enlèvement du premier moyen de fermeture et introduction de l'échantillon biologique dans le récipient.

Ainsi, grâce à ce dispositif, l'utilisateur enlève d'abord le moyen d'obturation de l'ouverture pour y introduire l'échantillon de prélèvement puis, il referme cette ouverture à l'aide du second moyen d'obturation qui reste en quelque sorte en réserve à l'autre extrémité du dispositif. Le dispositif peut ainsi être fermé par un moyen d'obturation stérile, la stérilité des faces internes du moyen d'obturation au niveau de l'ouverture ayant été assurée par le premier moyen d'obturation.

Cette invention trouvera donc son application en biologie médicale : coproculture mais surtout examen cytobactériologique des urines et ce dernier aspect sera développé en priorité.

Suivant une autre caractéristique, le premier moyen de fermeture est un opercule et/ou un capuchon et le second moyen de fermeture est un capuchon.

Lorsque le premier moyen de fermeture est un opercule, celui-ci peut être protégé sur le plan bactériologique et sur le plan mécanique, de manière complémentaire par un capuchon.

Suivant une autre caractéristique, l'extrémité supérieure et l'extrémité inférieure sont munies d'un filetage identique et les capuchons supérieur et inférieur sont munis d'un filetage correspondant pour être vissés l'un, sur le filetage de l'extrémité supérieure, l'autre, sur le filetage de l'extrémité inférieure en position d'attente puis sur le filetage de l'extrémité supérieure après enlèvement du premier capuchon et introduction de l'échantillon biologique.

Bien que le capuchon puisse être simplement emmanché sur le flacon, il est intéressant de le visser comme dans le mode de réalisation conforme aux caractéristiques ci-dessus.

Afin de mieux préserver la stérilité des faces internes de ce bouchon supplémentaire, la paroi du flacon comporte sur l'ensemble de son périmètre, un renforcement en dehors, surplombant le filetage inférieur, constituant ainsi une collerette, elle-même creusée sur sa face inférieure d'une gouttière destinée à recevoir le bord libre du bouchon sur toute sa circonférence.

Toujours dans le but de préserver la stérilité de ce bouchon "inférieur", la paroi du flacon se prolonge vers le bas et de quelques millimètres, au-delà du fond circulaire du récipient.

Suivant une autre caractéristique, le flacon est cylindrique à section circulaire.

Pour faciliter la préhension, il est intéressant que le flacon comporte un manche et que celui-ci soit notamment percé en son milieu d'une large fenêtre.

Le manche peut être fixé solidairement au flacon ou de manière amovible par une liaison à crochet et fenêtre.

Suivant une autre caractéristique, la paroi du flacon se prolonge vers le bas au-delà du fond et constitue un bord libre circulaire d'un cul de sac cylindrique inférieur et le fond du flacon est garni d'un produit bactériostatique.

Ainsi, à titre d'exemple, le flacon comporte sur sa paroi, aux 2/3 environ de sa hauteur, un crochet à angle droit ouvert en bas, permettant d'accepter un manche plat transversalement percé d'une fente à son extrémité proximale et sur la surface plane, le manche est longitudinalement percé d'une large fenêtre afin d'éviter à d'éventuelles gouttes d'urines d'atteindre les doigts du manipulateur.

Ainsi, l'avantage de cette invention consiste à disposer d'un flacon stérile :
- dont l'ouverture est initialement et provisoirement close hermétiquement par un moyen d'obturation stérile qui ne servira plus à refermer le flacon plein, car risquant fort d'avoir été contaminé pendant le prélèvement. Ce moyen sera donc jetable après ouverture du flacon ;
- dont la fermeture définitive après remplissage s'effectuera par un autre moyen obturateur stérile dégagé rapidement de la partie inférieure où il était jusqu'alors fixé, et qui n'aura donc subi aucun risque de contamination.

L'ensemble étant muni d'un manche permettant de tenir éloignée la main tenant le flacon des bords libres stériles de celui-ci.

Ainsi, lors du prélèvement urinaire, le patient :
- tient le flacon par l'extrémité de son manche,
- l'ouvre, et jette le bouchon qui obstruait initialement le flacon,
- récolte l'échantillon urinaire,
- dégage rapidement, une fois le flacon plein, l'autre bouchon stérile de la partie inférieure, pour immédiatement et sans le lâcher, s'en servir pour fermer définitivement le flacon.

Tous les risques de contamination de l'échantillon urinaire énumérés plus haut ont été ainsi évités.

Pour le prélèvement des selles, le même procédé sera utilisé avec surtout un flacon plus large et un manche rigide résistant et plus long.

Une forme simple et préférentielle de réalisation de l'invention est décrite ci-après en référence aux dessins annexés dans lesquels :
    - la figure 1 représente le dispositif selon l'invention, en coupe longitudinale et le manche en perspective,
    - la figure 2 est une vue en coupe d'une variante de l'extrémité inférieure du dispositif selon une variante.

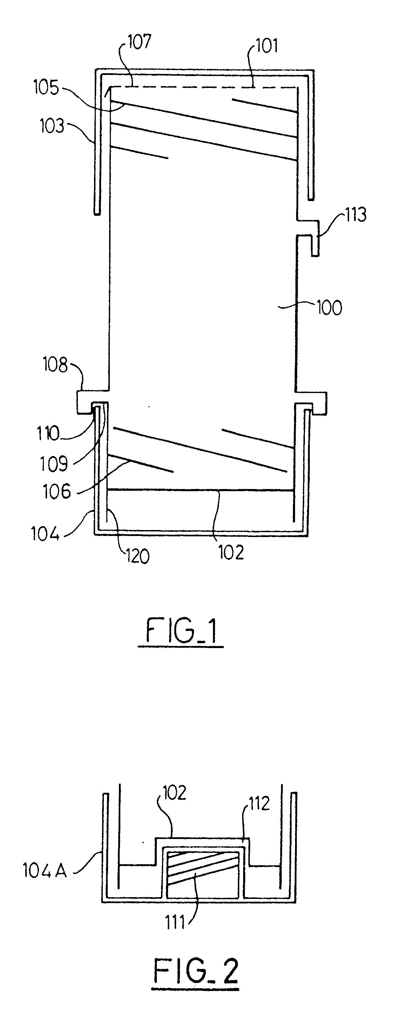

Selon le mode de réalisation de la figure 1, l'invention concerne un dispositif destiné à faciliter le prélèvement urinaire, tout en éliminant les risques de contamination de l'échantillon biologique ainsi recueilli. Ce dispositif se compose d'un flacon 100 à extrémité supérieure 101 ouverte et à fond 102 fermé. L'extrémité supérieure ouverte 101 est coiffée par un premier moyen d'obturation 103 en forme de capuchon et l'extrémité inférieure 102 est coiffée par un second moyen d'obturation 104 qui est en situation protégée et en attente. Selon l'exemple de la figure 1, les deux capuchons 103, 104 sont munis intérieurement d'un filetage non représenté destiné à coopérer avec le filetage 105 de l'extrémité supérieure et le filetage 106 de l'extrémité inférieure.

Selon une variante, l'extrémité supérieure est fermée par un opercule 107 détachable, recouvert ou non du capuchon 103. Dans ce cas, cet opercule constitue avec ou sans le capuchon 103, le premier moyen d'obturation.

A la figure 1, l'extrémité inférieure est prolongée au-delà du fond 102 par un bord libre circulaire 120 formant un cul de sac inférieur destiné à parfaire l'étanchéité et assurer la parfaite stérilité du bouchon.

Pour l'utilisation, on enlève le premier moyen d'obturation, c'est-à-dire le capuchon 103 et/ou l'opercule 107 pour faire le prélèvement puis on utilise le capuchon 104 qui était jusqu'alors en situation protégée, pour refermer le flacon.

Selon l'invention, le flacon 100 peut avoir une forme quelconque ; de manière générale, il s'agit d'un flacon cylindrique à section circulaire. Ce flacon peut être réalisé en une matière plastique rigide, plus ou moins souple et ayant une capacité d'environ 40 ml.

Selon la figure 1, le capuchon 104 vient sous un renforcement externe 108 constituant une collerette et dont la face inférieure est creusée d'une gorge 109 qui reçoit le bord supérieur 110 du capuchon 104 et le protège.

Selon l'invention, dans le cas où les capuchons 103 et 104 sont fixés par vissage, leurs filetages sont les mêmes et les filetages 105 et 106 sont également identiques.

Pour distinguer les capuchons ou plus généralement les moyens d'obturation 103 et 104, ceux-ci ont une couleur différente.

Suivant une variante non représentée, la membrane ou opercule 107 qui ferme l'ouverture 101 supérieure du flacon 100 n'est pas recouverte par un capuchon ; cet opercule est simplement fixé par collage ou d'une autre manière sur le bord libre de l'ouverture 101.

Suivant une autre variante non représentée, les moyens d'obturation ou capuchons 103, 104 sont simplement engagés par friction sur l'extrémité correspondante du flacon ou encore par enclipsage.

La figure 2 montre une autre variante de fixation du capuchon inférieur 104A qui comporte un bossage central 111 fileté venant se visser dans la cavité 112 du fond 102 du flacon partiellement représenté.

Selon l'invention, le flacon 100 est muni d'une poignée ou d'un manche solidaire, amovible ou rabattable. Dans l'exemple de réalisation représenté à la figure 1, ce manche est adaptable et, à cet effet, le flacon 100 comporte un crochet 113 recourbé vers le bas et le manche adaptable 114 comporte un bouclier 115 avant, en forme de segment de cylindre circulaire ou plus généralement de forme correspondant à la forme de la paroi du flacon 100 et prolongé par une languette 116 munie à l'avant d'une fente 117 pour recevoir le crochet 113 et à l'arrière d'une fente ou fenêtre importante et allongée 118 sur toute la longueur de la patte 116.

Ce manche peut également être rabattable, par exemple par l'intermédiaire d'une charnière.

Selon d'autres variantes non décrites, le capuchon inférieur peut être fixé par une collerette de papier ou autre moyen adhésif placé à cheval sur le bord du capuchon et la collerette 107.

Suivant une autre variante, il est également prévu une collerette supérieure non représentée dans laquelle vient se loger le bord inférieur du capuchon supérieur 103 toujours dans un but de protection contre la contamination.

Enfin, il peut être intéressant d'introduire dans le flacon un produit bactériostatique pour empêcher la multiplication des germes en cas de souillure.

## Revendications

1°) Dispositif destiné à faciliter le prélèvement urinaire tout en éliminant les risques de contamination de l'échantillon biologique ainsi recueilli, dispositif caractérisé en ce qu'il se compose d'un flacon (100) à extrémité supérieure ouverte (101), coiffée par un premier moyen de fermeture (103, 107) et un fond fermé (102), coiffé par un second moyen de fermeture (104), le second moyen de fermeture (104) étant destiné à coiffer l'extrémité supérieure ouverte (101) après l'enlèvement du premier moyen de fermeture (103, 107) et introduction de l'échantillon biologique dans le flacon (100).

2°) Dispositif selon la revendication 1, caractérisé en ce que le premier moyen de fermeture est un opercule (107) et/ou un capuchon (103) et le second moyen de fermeture (104) est un capuchon.

3°) Dispositif selon les revendications 1 et 2, caractérisé en ce que l'extrémité supérieure et l'extrémité inférieure sont munies d'un filetage identique (105, 106) et les capuchons supérieur (103) et inférieur (104) sont munis d'un filetage correspondant pour être vissés l'un, sur le filetage (105) de l'extrémité supérieure, l'autre, sur le filetage (106) de l'extrémité inférieure, en position d'attente puis sur le filetage de l'extrémité supérieure après enlèvement du premier capuchon (103) et introduction de l'échantillon biologique.

4°) Dispositif selon la revendication 1, caractérisé en ce que le flacon (100) comporte une collerette externe (108) creusée d'une gorge (109) au niveau de l'extrémité inférieure pour recouvrir le bord (110) du capuchon inférieur (104) lorsque ce dernier coiffe l'extrémité inférieure du flacon (100).

5°) Dispositif selon la revendication 1, caractérisé en ce que le flacon (100) est cylindrique à section circulaire.

6°) Dispositif selon la revendication 1, caractérisé en ce que le flacon est muni d'un manche (116).

7°) Dispositif selon la revendication 6, caractérisé en ce que le manche (116) est percé en son milieu d'une large fenêtre (118).

8°) Dispositif selon la revendication 6, caractérisé en ce que le manche (116) est adaptable sur un crochet (113) équipant le flacon (100) et comporte à cet effet une fente transversale (117) et un bouclier (115) applicable contre la paroi du flacon (100).

9°) Dispositif selon la revendication 1, caractérisé en ce que la paroi du flacon (100) se prolonge vers le bas au-delà du fond (102) et constitue un bord libre (120) circulaire d'un cul de sac cylindrique inférieur et le fond du flacon est garni d'un produit bactériostatique.

10°) Dispositif selon l'une quelconque des revendications 1 à 9, caractérisé en ce que le flacon, les capuchons et le manche sont réalisés en matière plastique.

FIG_1

FIG_2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 046 138  (LIBMAN et al.)<br>* Colonne 3, lignes 28-52; figures * | 1,2,5 | A 61 B   10/00 |
| Y | | 6-10 | |
| | --- | | |
| Y | US-A-4 244 920  (MANSCHOT et al.)<br>* Colonne 5, lignes 11-13,26-31;<br>figures * | 6-10 | |
| | --- | | |
| X | US-A-3 900 019  (LOGIADIS)<br>* Colonne 3, lignes 39-48; figures 2,4<br>* | 1-3,5 | |
| | --- | | |
| A | US-A-4 428 384  (RAITTO)<br>* Colonne 2, lignes 35-55; colonne 3,<br>ligne 53 - colonne 4, ligne 30; figures<br>* | 1,4 | |
| | --- | | |
| A | US-A-4 064 760  (BENJAMIN)<br>* Colonne 3, lignes 2-5; figures 9,10 * | 1,6-8 | |
| | --- | | |
| A | US-A-3 878 571  (SEELEY)<br>--- | | DOMAINES TECHNIQUES RECHERCHES (Int. Cl.4) |
| A | GB-A-1 312 447  (PIRT)<br>----- | | A 61 B<br>A 61 J<br>C 21 M |

Le présent rapport a été établi pour toutes les revendications

| Lieu de la recherche | Date d'achèvement de la recherche | Examinateur |
|---|---|---|
| LA HAYE | 25-07-1989 | GLAS J. |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
........................................................
& : membre de la même famille, document correspondant

EPO FORM 1503 03.82 (P0402)